# EUROPEAN PATENT APPLICATION

(11) **EP 3 023 421 A1**
(43) Date of publication of application: **25.05.2016**
(21) Application number: 14194323.3
(22) Date of filing: 21.11.2014
(51) Int. Cl.: C07D 405/12

(54) **Crystalline forms of afatinib dimaleate**

(71) Applicant: SANDOZ AG, 4056 Basel (CH)
(72) Inventor: The designation of the inventor has not yet been filed
(74) Representative: Schust, Jochen

(57) **Abstract**

A crystalline hydrate form of afatinib dimaleate of formula (I) having an X-ray powder diffraction pattern comprising reflections at at least four 2-theta values of (3.9 ± 0.2) °, (6.3 ± 0.2) °, (7.9 ± 0.2) °, (13.4 ± 0.2) °, (15.1 ± 0.2) °, (25.4 ± 0.2) °, when measured at a temperature in the range of from 15 to 25 °C with Cu-Kalpha_{1,2} radiation having a wavelength of 0.15419 nm.

## Description

### Field of Industrial Applicability

The present disclosure generally relates to polymorphic forms of the dimaleate salt of afatinib ("compound 1") and to processes for the preparation of these polymorphic forms. The present disclosure also generally relates to a pharmaceutical composition comprising the forms, as well of methods of using the form(s) in the treatment of disorders associated with pathological cellular proliferation, such as neoplasms, and cancer.

### Background of the Disclosure

The compound afatinib is also known under the trade name Gilotrif in the US and Giotrif in Europe, previously Tomtovok and Tovok, and was previously designated as BIBW-2992. It is a drug approved in United States, Europe, Taiwan, Mexico, Chile and Japan as well as other countries for the first-line treatment of patients with distinct types of metastatic (EGFR mutation positive) non-small cell lung carcinoma (NSCLC), developed by Boehringer Ingelheim. Afatinib has been given a Marketing Authorisation in the USA on 12 July 2013. It acts as an irreversible covalent inhibitor of the receptor tyrosine kinases epidermal growth factor receptor (EGFR) and erbB-2 (HER2).

The compound was also designated as "N-[4-[(3-Chloro-4-fluorophenyl)amino]-7-[[(3S)-tetrahydro-3-furanyl]oxy]-6-quinazolinyl]-4(dimethylamino)-2-butenamide", depicted below (as its dimaleate salt):

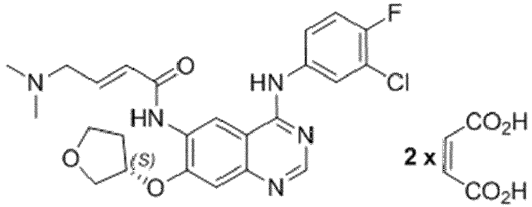

Like lapatinib and neratinib, afatinib is an irreversible inhibitor of the ErbB family of receptor tyrosine kinases (TKI). It covalently binds to and blocks signalling from all homo- and heterodimers formed by the ErbB family members EGFR (ErbB1), HER2 (ErbB2), ErbB3 and ErbB4. Aberrant ErbB signalling triggered by receptor mutations, and/or amplification, and/or receptor ligand overexpression contributes to the malignant phenotype. The recommended dose is 40 mg once daily.

Lung cancer is the leading cause of cancer-related mortality worldwide with an estimated 1 million newly diagnosed cases and 880 000 deaths each year. Non-small cell lung cancer (NSCLC) accounts for approximately 85% of all lung cancer cases. Progress has been made in the clinical management of early stage NSCLC by establishing comprehensive, multi-modality treatment regimens; however, the prognosis for advanced disease has not improved substantially. With an overall 5-year survival rate of 9% to 13%, the treatment of NSCLC remains a highly unmet medical need. In Europe, afatinib as monotherapy is indicated for the treatment of EGFR TKI-naïve adult patients with locally advanced or metastatic non-small cell lung cancer with activating EGFR mutation(s). Afatinib is under development for treatment of several solid tumors including non-small cell lung cancer (NSCLC), breast, head and neck cancer, and a variety of other cancers.

WO 2002/50043 and WO 2005/037824 disclose afatinib, salts thereof and a crystalline form of the di-maleate salt (also designated as Form A). Equivalent US 8,426,586 is cited in the FDA Orange Book for afatinib dimaleate.

Polymorphism, the occurrence of different crystal forms, is a property of some molecules and molecular complexes. A single molecule may give rise to a variety of polymorphs having distinct crystal structures and physical properties like melting point, thermal behaviors (e.g. measured by thermogravimetric analysis - "TGA", or differential scanning calorimetry - "DSC"), X-ray powder diffraction (XRPD or powder XRD) pattern, infrared absorption fingerprint, and solid state nuclear magnetic resonance (NMR) spectrum. One or more of these techniques may be used to distinguish different polymorphic forms of a compound.

New polymorphic forms and solvates of a pharmaceutical product can provide materials having desirable processing properties, such as ease of handling, ease of processing, storage stability, ease of purification or as desirable intermediate crystal forms that facilitate conversion to other polymorphic forms. New polymorphic forms and solvates of a pharmaceutically useful compound or salts thereof can also provide an opportunity to improve the performance characteristics of a pharmaceutical product. It enlarges the repertoire of materials that a formulation scientist has available for formulation optimization, for example by providing a product with different properties, e.g., better processing or handling characteristics, improved dissolution profile, improved stability, improved purity, or improved shelf-life.

During examination of EP 2 508 521 (which derived from WO 2005/037824), the respective applicant filed a report on polymorphic forms and solvates of the dimaleate salt dated 11 July 2013, wherein it was stated that for pharmaceutical use an active substance not only has to exhibit the desired pharmacological activity and tolerability but must also fulfill a range of physicochemical qualities to be suitable for use of a medicament, i.e.:
- The compound must be stable over a long period under the temperature and humidity conditions which apply in the different climate zones, in order to prevent dose reduction via decomposition as well decomposition products causing adverse effects to the patients.
- The active must occur in a stable and uniform crystalline form, in order to prevent that conversion of the crystalline form into another leads to content unity changes of the (tablet) formulation or even destruction of the formulation.
- Another criterion of exceptional importance is the solubility of the active substance. Oral drugs, as is the in the subject invention, should be sufficiently soluble to allow high release of the active in the gastrointestinal tract, good resorption and high bioavailability.

It is further stated in this report that the dimaleate salt of afatinib is highly crystalline but shows a very uncomplicated polymorphism, i.e.
- an anhydrous form A obtained by crystallisation in the absence or presence of moderate amounts of water
- a dihydrate form B obtained by crystallisation in the presence of high excess of water such as under evaporating conditions
- a monohydrate form C (metastable, turning into stable Form A above 100°C)
- a hexahydrate form D (metastable).

The respective applicant points out that the anhydrous form A of the dimaleate is very much preferred for processing into tablets since hydrolytic decomposition of the active agent is a critical issue and the presence of water should thus be avoided in pharmaceutical tablet formulations. In particular, it is emphasized in the report that the hydrate forms of afatinib are therefore not suitable for the production of the active agent to be processed into final solid formulations.

Further salts and crystalline forms of afatinib are described in WO 2012/121764 (disclosing an afatinib dimaleate "Form B") and WO 2013/052157 (disclosing afatinib dimaleate "Forms C, D and E"). Form E as described in WO 2013/052157 appears to be hydrate, but does not appear to show a stable and uniform crystalline form (according to the XRPD).

Hence, although it may appear that many additional different solid state forms of afatinib dimaleate can be produced, only very few of the additional solid state forms show desirable characteristics for a pharmaceutical product.

Typically, afatinib dimaleate is administered orally, as this route provides comfort and convenience of dosing. Although salts of afatinib and polymorphic forms thereof are known in the art, finding a good or even the optimal form with regard to bioavailability, inter-patient variability, and safety remains a considerable challenge, in particular when the compound forms many salts or polymorphic forms. Further, not all physical forms of afatinib are equally suitable with regard to polymorphic and chemical stability, flow properties, compressibility, dissolution rate, and some are at least to some extent hygroscopic or show electrostatic charging. These properties can constitute disadvantages in the preparation of pharmaceutical compositions, such as tablets.

It is therefore an object of the present invention to provide crystalline forms of afatinib dimaleate, as well as pharmaceutical compositions comprising the same, which do not show at least one or more of the above-described problematic properties. In particular, it is an object to provide crystalline forms of afatinib dimaleate which show good bioavailability, low inter-patient variability, excellent overall therapeutic efficacy, excellent polymorphic and/or chemical stability, excellent flow properties, good compressibility, an excellent dissolution profile, and which are non-hygroscopic and/or do not electrostatically charge. The present inventors have found crystalline forms of afatinib dimaleate which shows advantageous properties in at least one of the mentioned aspects.

Despite the above-described prejudice in the art, it has surprisingly been found that hydrate forms of afatinib dimaleate can be provided which have at least one the following properties:
- are stable over a long period under the temperature and humidity conditions which apply in the different climate zones
- occur in a stable and uniform crystalline form
- are sufficiently soluble to allow high release of the active in the gastrointestinal tract, good resorption and high bioavailability.

### Summary of the Invention

Therefore, the present invention relates to a crystalline hydrate form of afatinib dimaleate of formula (I) 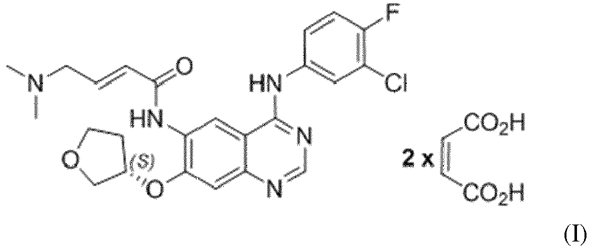 having an X-ray powder diffraction pattern comprising reflections at at least four 2-theta values of (3.9 ± 0.2) °, (6.3 ± 0.2) °, (7.9 ± 0.2) °, (13.4 ± 0.2) °, (15.1 ± 0.2) °, (25.4 ± 0.2) °, when measured at a temperature in the range of from 15 to 25 °C, preferably at 22 °C, with Cu-Kalpha_{1,2} radiation having a wavelength of 0.15419 nm. In the context of the present disclosure, this crystalline hydrate form is also referred to as **"Form 12".**

Further, the present invention relates to a crystalline hydrate form of afatinib dimaleate of formula (I) having an X-ray powder diffraction pattern comprising reflections at 2-theta values of (5.5 ± 0.2) °, (9.8 ± 0.2) °, (11.7 ± 0.2) °, (14.9 ± 0.2) °, when measured at a temperature in the range of from 15 to 25 °C, preferably at 22 °C, with Cu-Kalpha_{1,2} radiation having a wavelength of 0.15419 nm. In the context of the present disclosure, this crystalline hydrate form is also referred to as **"Form 4".**

Further, the present disclosure describes a crystalline hydrate form of afatinib dimaleate of formula (I) having an X-ray powder diffraction pattern comprising reflections at 2-theta values of (5.3 ± 0.2) °, (10.2 ± 0.2) °, (10.7 ± 0.2) °, when measured at a temperature in the range of from 15 to 25 °C, preferably at 22 °C, with Cu-Kalpha_{1,2} radiation having a wavelength of 0.15419 nm. In the context of the present disclosure, this crystalline hydrate form is also referred to as **"Form 3".**

Further, the present disclosure describes a crystalline hydrate form of afatinib dimaleate of formula (I) having an X-ray powder diffraction pattern comprising reflections at at least four 2-theta values of (5.1 ± 0.2) °, (10.1 ± 0.2) °, (13.9 ± 0.2) °, (15.2 ± 0.2) °, (20.4 ± 0.2) °, when measured at a temperature in the range of from 15 to 25 °C, preferably at 22 °C, with Cu-Kalpha_{1,2} radiation having a wavelength of 0.15419 nm. In the context of the present disclosure, this crystalline hydrate form is also referred to as **"Form 7".**

### Brief Description of the Figures

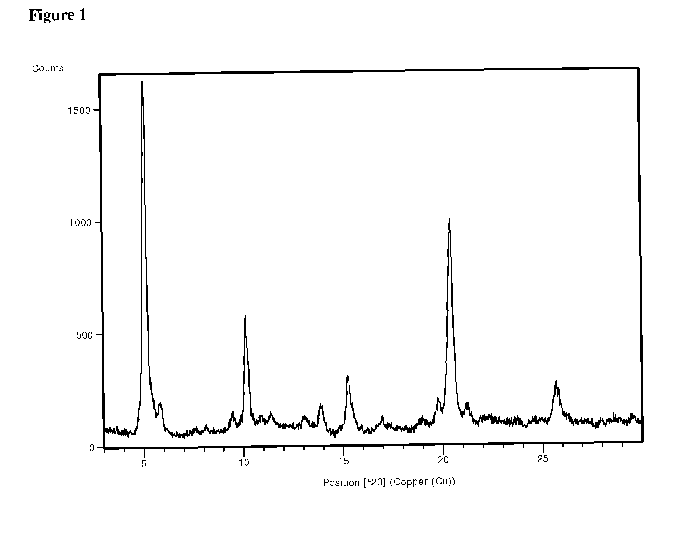

- Fig. 1.: illustrates the x-ray powder diffraction pattern of crystalline form 7 of afatinib dimaleate as obtained according to Reference Example 4. The x axis shows the 2 Theta position in ° with tick marks, from left to right, at 5, 10, 15, 20, 25. The y axis shows the intensity in counts, with tick marks, from bottom to top, at 0, 500, 1000, 1500.

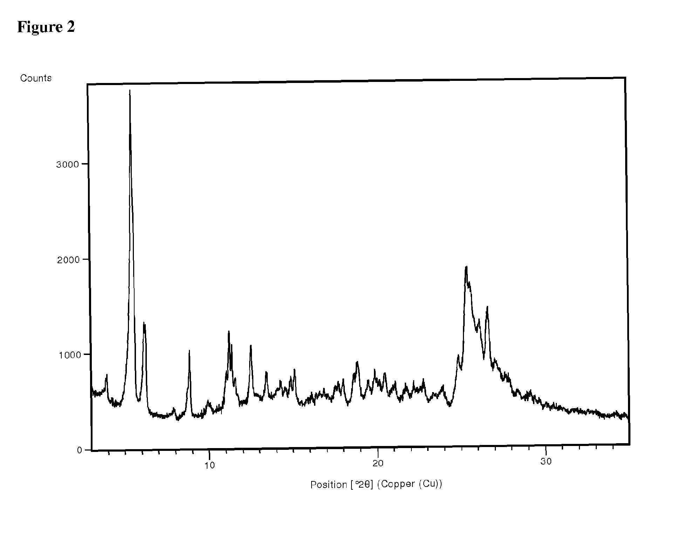

- Fig. 2.: illustrates the x-ray powder diffraction pattern of crystalline form 12 of afatinib dimaleate as obtained according to Example 1. The x axis shows the 2 Theta position in ° with tick marks, from left to right, at 10, 20, 30. The y axis shows the intensity in counts, with tick marks, from bottom to top, at 0, 1000, 2000, 3000.

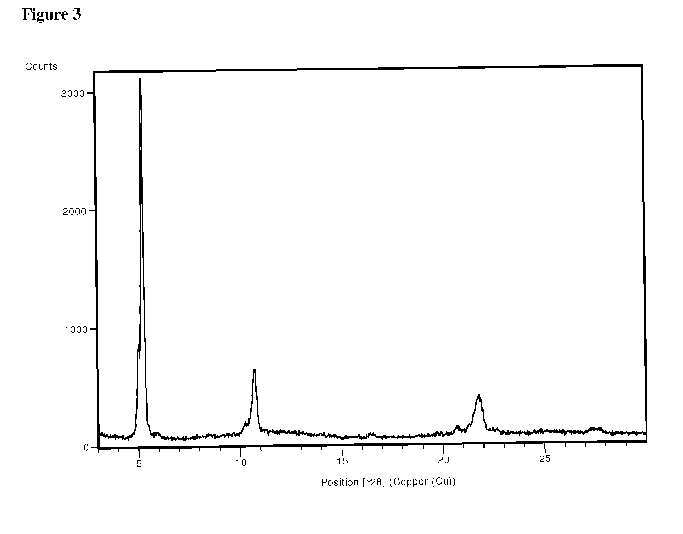

- Fig. 3.: illustrates the x-ray powder diffraction pattern of crystalline form 3 of afatinib dimaleate as obtained according to Example 3. The x axis shows the 2 Theta position in ° with tick marks, from left to right, at 5, 10, 15, 20, 25. The y axis shows the intensity in counts, with tick marks, from bottom to top, at 0, 1000, 2000, 3000.

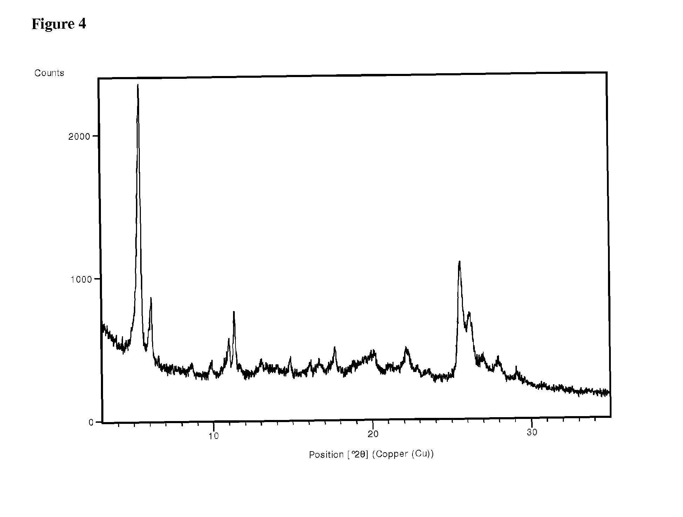

- Fig. 4.: illustrates the x-ray powder diffraction pattern of crystalline form 4 of afatinib dimaleate as obtained according to Example 4. The x axis shows the 2 Theta position in ° with tick marks, from left to right, at 10, 20, 30. The y axis shows the intensity in counts, with tick marks, from bottom to top, at 0, 1000, 2000.

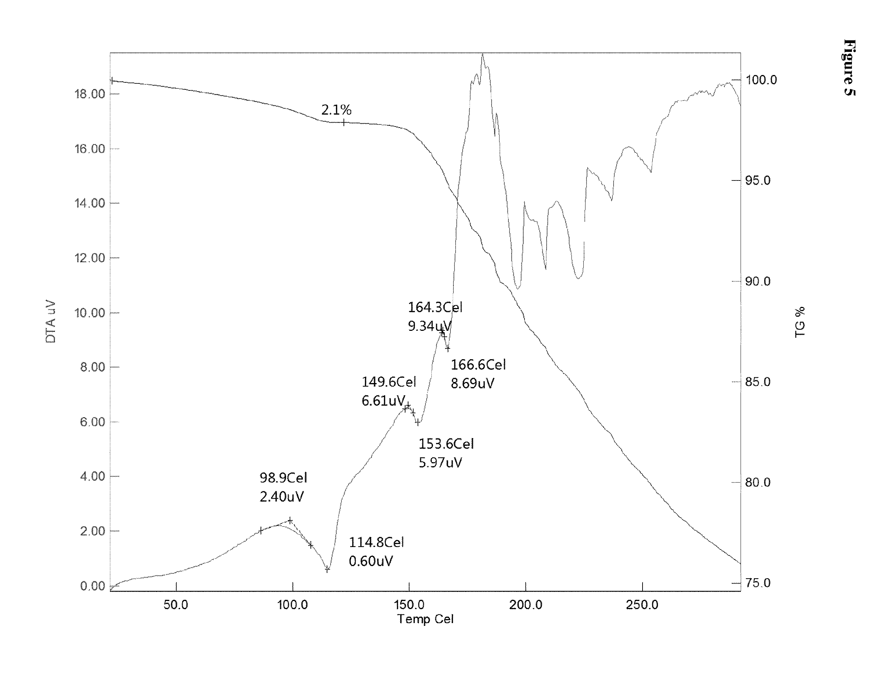

- Fig. 5: illustrates the TG/DTA curves of crystalline form 12 of afatinib dimaleate as obtained according to Example 1, determined as described in Reference Example 1.4.

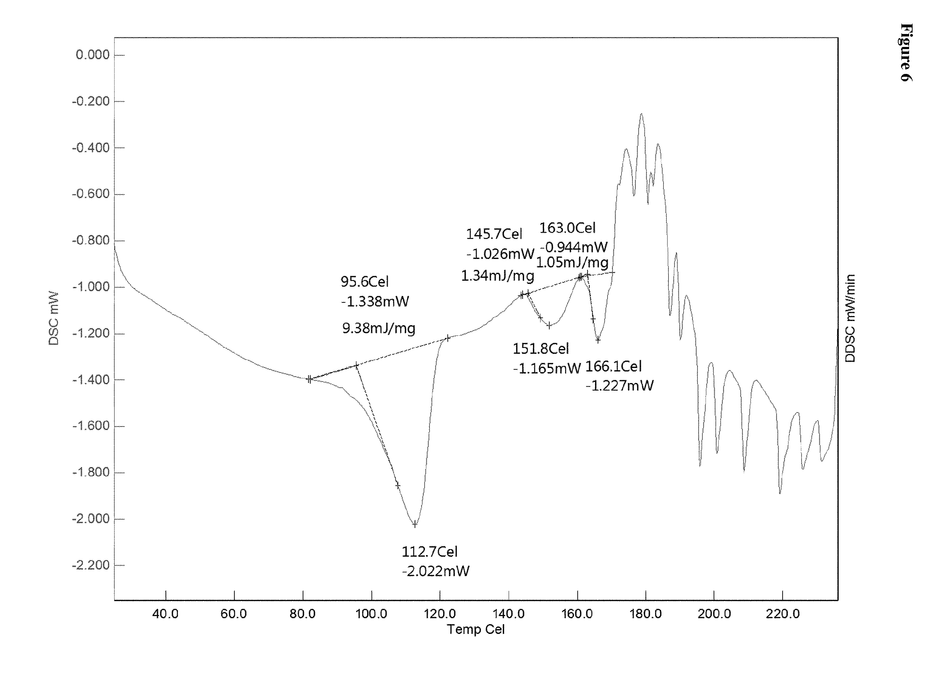

- Fig. 6: illustrates the DSC curves of crystalline form 12 of afatinib dimaleate as obtained according to Example 1, determined as described in Reference Example 1.3.

### Detailed Description of the Invention

The disclosure relates to the crystalline hydrate forms of afatinib dimaleate mentioned above, which are described and characterized in detail herein. Further, preferred processes for the preparation of these crystalline hydrates are described.

### Definitions

As used herein "polymorph" refers to crystalline forms having the same chemical composition but different spatial arrangements of the molecules, atoms, and/or ions forming the crystal.

As used herein "solvate" refers to a crystalline form of a molecule, atom, and/or ions that further comprises molecules of a solvent or solvents incorporated into the crystalline lattice structure. The solvent molecules in the solvate may be present in a regular arrangement and/or a non-ordered arrangement. The solvate may comprise either a stoichiometric or non-stoichiometric amount of the solvent molecules. For example, a solvate with a nonstoichiometric amount of solvent molecules may result from partial loss of solvent from the solvate. Solvates may occur as dimers or oligomers comprising more than one molecule of afatinib within the crystalline lattice structure.

As used herein "amorphous" refers to a solid form of a molecule, atom, and/or ions that is not crystalline. An amorphous solid does not display a definitive X-ray diffraction pattern.

As used herein, the term "substantially pure" with reference to a particular polymorphic form means that the polymorphic form includes less than 10%, preferably less than 5%, more preferably less than 3%, most preferably less than 1% by weight of any other physical forms of the compound.

### Form 12

As mentioned above, the crystalline hydrate form 12 of afatinib dimaleate of formula (I) has an X-ray powder diffraction pattern comprising reflections at at least four 2-theta values of (3.9 ± 0.2) °, (6.3 ± 0.2) °, (7.9 ± 0.2) °, (13.4 ± 0.2) °, (15.1 ± 0.2) °, (25.4 ± 0.2) °. Preferably, the X-ray powder diffraction pattern comprises reflections at at least five, preferably at least six 2-theta values of (3.9 ± 0.2) °, (6.3 ± 0.2) °, (7.9 ± 0.2) °, (13.4 ± 0.2) °, (15.1 ± 0.2) °, (25.4 ± 0.2) °. More preferably, the X-ray powder diffraction pattern comprises reflections at 2-theta values of (3.9 ± 0.2) °, (6.3 ± 0.2) °, (7.9 ± 0.2) °, (13.4 ± 0.2) °, (15.1 ± 0.2) °, (25.4 ± 0.2) °. Preferably, in addition to the peaks mentioned above, the X-ray powder diffraction pattern of form 12 comprises at least one peak at a 2-theta value (5.5 ± 0.2) °, (11.3 ± 0.2) °, (12.5 ± 0.2) °.

The crystalline hydrate form 12 can be further characterized by thermal analysis via DTA which indicated three thermal events, the onsets of which varied only very slightly at the different stages of drying. In particular, the crystalline hydrate form 12 can be further characterized by thermal analysis via DTA by a broad endotherm at an onset of about 100 °C and a peak at about 114 °C, a small endotherm at an onset of about 150 °C and a peak at about 155 °C, and a small endotherm at an onset of about 165 °C and a peak at about 167 °C.

The crystalline hydrate form 12 can be further characterized by thermal analysis via DSC which indicated three thermal events, the onsets of which varied only very slightly at the different stages of drying. In particular, the crystalline hydrate form 12 can be further characterized by thermal analysis via DSC by a broad endotherm at an onset of about 95.6 °C and a peak at about 112.7 °C, a broad endotherm at an onset of about 145.7 °C and a peak at about 151.8 °C, and a sharp endotherm at an onset of about 163.0 °C and a peak at about 166.1 °C.

The crystalline hydrate form 12 can be further characterized by its water content which, as determined by Karl Fischer tritration, is preferably in the range of from 4.8 to 5.0 weight-%, based on the total weight of the crystalline form 12.

A preferred process for preparing the crystalline hydrate form 12 comprises providing solid afatinib dimaleate of formula (I) in crystalline form 7 (see characterization herein below) and subjecting this crystalline form to specific drying conditions, preferably to drying at a temperature in the range of from 15 to 30 °C at an absolute pressure of at most 500 mbar for 1 to 24 hours, more preferably to drying at a temperature in the range of from 20 to 25 °C at an absolute pressure of at most 250 mbar, preferably of at most 100 mbar, for 6 to 24 hours, preferably for 8 to 16 hours. From this specific drying, the crystalline hydrate form 12 is obtained.

Preferably, the non-dried form 7 is prepared from solid afatinib dimaleate of formula (I) in crystalline form 1 (having an X-ray powder diffraction pattern as described in the Table on page 11 and Figure 1 of WO 2005/037824, which form is referred to herein also as "form A"). With regard to a process for the preparation of form 1, reference can be made, for example, to WO 2005/037824. According to this process via form 1, it is preferred that form 1 as provided is dissolved in a solvent comprising a ketone, preferably comprising acetone. Preferably, at least 99 weight-% of the solvent, more preferably at least 99.5 weight of the solvent consist of acetone. The respectively obtained solution is preferably suitably cooled wherein during cooling, form 7 crystallizes and is obtained as solid material suspended in its mother liquor. From this suspension, the crystalline form 7 is preferably suitably separated, preferably by filtration, before it is subjected to the specific drying as described above. Preferred characteristics of the process are described in detail in the embodiment section of this disclosure.

An especially preferred process for preparing form 12 therefore comprises
(i) providing solid afatinib dimaleate of formula (I) in the crystalline form 7, wherein said providing comprises
   (i-1) providing solid afatinib dimaleate of formula (I) in crystalline form 1 (e.g., as provided in WO 2005/037824);
   (i-2) dissolving the solid afatinib dimaleate of formula (I) provided in (i-1) in acetone at a temperature in the range of from 22.5 to 27.5 °C;
   (i-3) cooling the solution obtained in (i-2) to a temperature in the range of from 7.5 to 12.5 °C, obtaining the solid afatinib dimaleate of formula (I) in the crystalline form 7 in its mother liquor;
   (i-4) separating the solid afatinib dimaleate of formula (I) obtained in (i-3) from its mother liquor by filtration;
(ii) subjecting the non-dried solid state afatinib dimaleate provided in (i) to drying at a temperature in the range of from 20 to 25 °C at an absolute pressure of at most 250 mbar, preferably of at most 100 mbar, for 6 to 24 hours, preferably for 8 to 16 hours, obtaining the crystalline form 12 of afatinib dimaleate of formula (I).

Also preferably, the non-dried form 7 is prepared from solid amorphous afatinib dimaleate of formula (I). With regard to a process for the preparation of solid amorphous afatinib dimaleate of formula (I), reference can be made, for example, to WO 2005/037824. According to this process, it is preferred that solid amorphous afatinib dimaleate as provided is slurried in a liquid medium which comprises water and an alcohol. Generally, it is conceivable that the liquid medium comprises two or more alcohols. Preferably, an alcohol which is comprised in the liquid medium is a propanol, such as n-propanol or isopropanol. More preferably, an alcohol which is comprised in the liquid medium is n-propanol. More preferably, from 65 to 95 weight-%, preferably from 75 to 85 weight-%, more preferably from 77.5 to 82.5 weight-% of the liquid medium consist of the alcohol, preferably the propanol, more preferably n-propanol, and from 5 to 35 weight-%, preferably from 15 to 25 weight-%, more preferably from 17.5 to 22.5 weight-% of the liquid medium consist of water. The respectively obtained suspension is preferably subjected to a temperature cycle between a minimum temperature T₁ and a maximum temperature T₂. In particular, the suspension prepared as described above is first brought to a temperature T₂, kept at this temperature for a certain period of time, then cooled to a minimum temperature T₁, kept at this temperature for a certain period of time, then heated to a temperature T₂, kept at this temperature for a certain period of time, and so forth. Preferably, at the end of the temperature cycle, the temperature is a temperature T₁. Preferred characteristics of the temperature cycle are described in detail in the embodiment section of this disclosure. During this temperature cycle, form 7 crystallizes and is obtained as solid material suspended in its mother liquor. From this suspension, the crystalline form 7 is preferably suitably separated, preferably by filtration, before it is subjected to the specific drying as described above. Preferred characteristics of the process are described in detail in the embodiment section of this disclosure.

Another especially preferred process for preparing form 12 therefore comprises
(i) providing solid afatinib dimaleate of formula (I) in crystalline form 7, wherein said providing comprises
   (i-1) providing solid amorphous afatinib dimaleate of formula (I);
   (i-2) slurrying the solid amorphous afatinib dimaleate of formula (I) provided in (i-1) in a liquid medium, wherein 75 to 85 weight-% of the liquid medium consist of n-propanol and from 15 to 25 weight-% of the liquid medium consist of water, at a temperature in the range of from 20 to 25 °C;
   (i-3) subjecting the slurry obtained in (i-2) to a temperature cycle between a minimum temperature T₁ and a maximum temperature T₂, wherein T₁ is in the range of from 2 to 7.5 °C and T₂ is in the range of from 37.5 to 42.5 °C, obtaining the solid afatinib dimaleate of formula (I) in crystalline form 7 in the liquid medium, wherein during the temperature cycle, the slurry is kept at T₁ for 3 to 5 h and at T₂ for 3 to 5 h, wherein the temperature cycle is carried out for an overall time in the range of from 48 to 76 hours, and wherein during the temperature cycle, the heating rate from T₁ to T₂ is in the range of from 0.75 to 1.25 °C/min and the cooling rate from T₂ to T₁ is in the range of from 0.75 to 1.25 °C/min;
   (i-4) separating the solid afatinib dimaleate of formula (I) obtained in (i-3) from the liquid medium by filtration;
(ii) subjecting the solid state afatinib dimaleate provided in (i) to drying at a temperature in the range of from 20 to 25 °C at an absolute pressure of at most 250 mbar, preferably of at most 100 mbar, for 6 to 24 hours, preferably for 8 to 16 hours, obtaining the crystalline form 12 of afatinib dimaleate of formula (I).

Another preferred process for preparing the crystalline hydrate form 12 comprises providing solid afatinib dimaleate of formula (I) of crystalline form 1 and slurrying form 1 in a liquid medium comprising a nitrile. Generally, it is conceivable that the liquid medium comprises two or more nitriles. Preferably, a nitrile which is comprised in the liquid medium is a acetonitrile. More preferably, at least 99 weight-%, more preferably at least 99.5 weight-% of the liquid medium consist of acetonitrile. The slurry is preferably agitated at elevated temperatures, for example stirred, wherein form 12 crystallizes and is obtained as solid material suspended in its mother liquor. From this suspension, the crystalline form 12 is preferably suitably separated, preferably by filtration, before it is subjected to drying. Preferred characteristics of the process are described in detail in the embodiment section of this disclosure.

An especially preferred process for preparing form 12 therefore comprises
(i) providing solid afatinib dimaleate of formula (I) in the crystalline form 1;
(ii) slurrying the solid afatinib dimaleate of formula (I) provided in (i) in acetonitrile at a temperature in the range of from 20 to 25 °C;
(iii) agitating, preferably stirring the slurry obtained in (iii) at a temperature in the range of from 47.5 to 52.5 °C for 60 to 78 hours;
(iv) separating the solid from the acetonitrile by filtration;
(v) subjecting the solid obtained from (iv) to drying at a temperature in the range of from 20 to 25 °C at an absolute pressure in the range of from 5mbar to 100mbar, obtaining the crystalline hydrate form 12 of afatinib dimaleate of formula (I).

### Form 4

As mentioned above, the crystalline hydrate form 4 of afatinib dimaleate of formula (I) has an X-ray powder diffraction pattern comprising reflections at 2-theta values of (5.5 ± 0.2) °, (9.8 ± 0.2) °, (11.7 ± 0.2) °, (14.9 ± 0.2) °. Preferably, in addition to the peaks mentioned above, the X-ray powder diffraction pattern of form 4 comprises one or more reflections at 2-theta values of (6.1 ± 0.2) °, (25.6 ± 0.2) °, preferably reflections at 2-theta values of (6.1 ± 0.2) °, (25.6 ± 0.2) °.

The crystalline hydrate form 4 can be further characterized by its water content which, as determined by Karl Fischer tritration, is preferably in the range of from 7.9 to 8.1 weight-%, based on the total weight of the crystalline form 4.

A preferred process for preparing the crystalline hydrate form 4 comprises providing solid amorphous afatinib dimaleate of formula (I) as described above, and slurrying the solid amorphous afatinib dimaleate in a liquid medium comprising an alcohol. Generally, it is conceivable that the liquid medium comprises two or more alcohols. Preferably, an alcohol which is comprised in the liquid medium is a propanol, such as n-propanol or isopropanol. More preferably, an alcohol which is comprised in the liquid medium is n-propanol. More preferably, at least 99 weight-%, preferably at least 99.5 weight-% of the liquid medium consist of the alcohol, preferably the propanol, more preferably n-propanol. The respectively obtained suspension is preferably subjected to a temperature cycle between a minimum temperature T₁ and a maximum temperature T₂. In particular, the suspension prepared as described above is first brought to a temperature T₂, kept at this temperature for a certain period of time, then cooled to a minimum temperature T₁, kept at this temperature for a certain period of time, then heated to a temperature T₂, kept at this temperature for a certain period of time, and so forth. Preferably, at the end of the temperature cycle, the temperature is a temperature T₁. Preferred characteristics of the temperature cycle are described in detail in the embodiment section of this disclosure. During this temperature cycle, form 4 crystallizes and is obtained as solid material suspended in its mother liquor. From this suspension, the crystalline form 4 is preferably suitably separated, preferably by filtration, before it is subjected to the specific drying as described above. Preferred characteristics of the process are described in detail in the embodiment section of this disclosure.

An especially preferred process for preparing form 4 therefore comprises
(i) providing solid amorphous afatinib dimaleate of formula (I);
(ii) slurrying the solid amorphous afatinib dimaleate of formula (I) provided in (i) in n-propanol, at a temperature in the range of from 20 to 25 °C;
(iii) subjecting the slurry obtained in (ii) to a temperature cycle between a minimum temperature T₁ and a maximum temperature T₂, wherein T₁ is in the range of from 2 to 7.5 °C and T₂ is in the range of from 37.5 to 42.5 °C, obtaining crystalline form 4 of afatinib dimaleate of formula (I) in the liquid medium, wherein during the temperature cycle, the slurry is kept at T₁ for 3 to 5 h and at T₂ for 3 to 5 h, wherein the temperature cycle is carried out for an overall time in the range of from 48 to 76 hours, and wherein during the temperature cycle, the heating rate from T₁ to T₂ is in the range of from 0.75 to 1.25 °C/min and the cooling rate from T₂ to T₁ is in the range of from 0.75 to 1.25 °C/min;
(iv) separating the crystalline afatinib dimaleate of formula (I) obtained in (iii) from the n-propanol;
(v) drying the separated crystalline afatinib dimaleate of formula (I) at a temperature in the range of from 20 to 25 °C at an absolute pressure in the range of from 5mbar to 100mbar.

### Form 3

As mentioned above, the crystalline hydrate form 3 of afatinib dimaleate of formula (I) has an X-ray powder diffraction pattern comprising reflections at 2-theta values of (5.3 ± 0.2) °, (10.2 ± 0.2) °, (10.7 ± 0.2) °. Preferably, in addition to the peaks mentioned above, the X-ray powder diffraction pattern of form 3 comprises one or more reflections at 2-theta values of (16.5 ± 0.2) °, (27.5 ± 0.2) °, preferably reflections at 2-theta values of (16.5 ± 0.2) °, (27.5 ± 0.2) °.

The crystalline hydrate form 3 can be further characterized by its water content which, as determined by Karl Fischer tritration, is preferably in the range of from 7.2 to 7.4 weight-%, based on the total weight of the crystalline form 3.

A preferred process for preparing the crystalline hydrate form 3 comprises providing solid amorphous afatinib dimaleate of formula (I), slurrying the solid amorphous afatinib dimaleate in a liquid medium comprising water and a ketone. Generally, it is conceivable that the liquid medium comprises two or more ketones. Preferably, a ketone which is comprised in the liquid medium is acetone. More preferably, from 40 to 60 weight-%, preferably from 45 to 55 weight-%, more preferably from 47.5 to 52.5 weight-% of the liquid medium consist of the ketone, preferably acetone, and from 60 to 40 weight-%, preferably from 55 to 45 weight-%, more preferably from 52.5 to 47.5 weight-% of the liquid medium consist of water. The respectively obtained suspension is preferably subjected to a temperature cycle between a minimum temperature T₁ and a maximum temperature T₂. In particular, the suspension prepared as described above is first brought to a temperature T₂, kept at this temperature for a certain period of time, then cooled to a minimum temperature T₁, kept at this temperature for a certain period of time, then heated to a temperature T₂, kept at this temperature for a certain period of time, and so forth. Preferably, at the end of the temperature cycle, the temperature is a temperature T₁. Preferred characteristics of the temperature cycle are described in detail in the embodiment section of this disclosure. During this temperature cycle, form 3 crystallizes and is obtained as solid material suspended in its mother liquor. From this suspension, the crystalline form 3 is preferably suitably separated, preferably by filtration, before it is subjected to the specific drying as described above. Preferred characteristics of the process are described in detail in the embodiment section of this disclosure.

An especially preferred process for preparing form 3 therefore comprises
(i) providing solid amorphous afatinib dimaleate of formula (I);
(ii) slurrying the solid amorphous afatinib dimaleate of formula (I) provided in (i) in a liquid medium of which from 45 to 55 weight-% consist of water and from 55 to 45 weight-% consist of acetone, at a temperature in the range of from 20 to 25 °C;
(iii) subjecting the slurry obtained in (ii) to a temperature cycle between a minimum temperature T₁ and a maximum temperature T₂, wherein T₁ is in the range of from 2 to 7.5 °C and T₂ is in the range of from 37.5 to 42.5 °C, obtaining crystalline form 7 of afatinib dimaleate of formula (I) in the liquid medium, wherein during the temperature cycle, the slurry is kept at T₁ for 3 to 5 h and at T₂ for 3 to 5 h, wherein the temperature cycle is carried out for an overall time in the range of from 48 to 76 hours, and wherein during the temperature cycle, the heating rate from T₁ to T₂ is in the range of from 0.75 to 1.25 °C/min and the cooling rate from T₂ to T₁ is in the range of from 0.75 to 1.25 °C/min;
(iv) separating the crystalline afatinib dimaleate of formula (I) obtained in (iii) from the liquid medium;
(v) drying the separated crystalline afatinib dimaleate of formula (I) at a temperature in the range of from 20 to 25 °C at an absolute pressure in the range of from 5mbar to 100mbar.

### Form 7

As mentioned above, the crystalline hydrate form 7 of afatinib dimaleate of formula (I) has an X-ray powder diffraction pattern comprising at at least four 2-theta values of (5.1 ± 0.2) °, (10.1 ± 0.2) °, (13.9 ± 0.2) °, (15.2 ± 0.2) °, (20.4 ± 0.2) °. Preferably, the X-ray powder diffraction pattern comprises reflections at 2-theta values of (5.1 ± 0.2) °, (10.1 ± 0.2) °, (13.9 ± 0.2) °, (15.2 ± 0.2) °, (20.4 ± 0.2) °. Preferably, in addition to the peaks mentioned above, the X-ray powder diffraction pattern of form 7 comprises a reflection at 2-theta value of (25.7 ± 0.2) °.

The (non-dried) crystalline hydrate form 7 was found to be a crystalline form which can be used, for example as an intermediate, for preparing other crystalline forms of afatinib dimaleate of formula (I), preferably other crystalline hydrate forms of afatinib dimaleate of formula (I). By way of example, the crystalline hydrate form 7 can be used for preparing the crystalline form 12 as described in the present disclosure wherein, in turn, the crystalline hydrate form 7 can be prepared, for example, from amorphous afatinib dimaleate of formula (I) or from anhydrous crystalline form 1 of afatinib dimaleate of formula (I) wherein said form 1 is also referred to as "form A". With regard to form A, reference is made to the respective discussion in the background section of the present disclosure. Preferably, form 7 is prepared as described above.

Preferably, the (non-dried) form 7 can be prepared from solid afatinib dimaleate of formula (I) in crystalline form 1 (having an X-ray powder diffraction pattern as described in WO 2005/037824, which form is referred to herein also as "form A"). Also preferably, the non-dried form 7 can be prepared from solid amorphous afatinib dimaleate of formula (I).

The following non-limiting embodiments and combination of embodiments as indicated by the respective dependencies and references are illustrative for the disclosure.
1. A crystalline hydrate form of afatinib dimaleate of formula (I), also referred to herein as form 12, 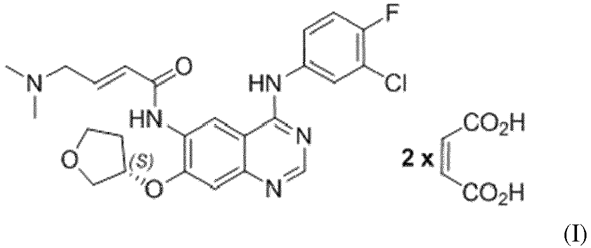 having an X-ray powder diffraction pattern comprising reflections at at least four 2-theta values of (3.9 ± 0.2) °, (6.3 ± 0.2) °, (7.9 ± 0.2) °, (13.4 ± 0.2) °, (15.1 ± 0.2) °, (25.4 ± 0.2) °, when measured at a temperature in the range of from 15 to 25 °C with Cu-Kalpha_{1,2} radiation having a wavelength of 0.15419 nm.
2. The crystalline hydrate form of embodiment 1, wherein the X-ray powder diffraction pattern comprises reflections at at least five, preferably at least six 2-theta values of (3.9 ± 0.2) °, (6.3 ± 0.2) °, (7.9 ± 0.2) °, (13.4 ± 0.2) °, (15.1 ± 0.2) °, (25.4 ± 0.2) °, when measured at a temperature in the range of from 15 to 25 °C with Cu-Kalpha_{1,2} radiation having a wavelength of 0.15419 nm.
3. The crystalline hydrate form of embodiment 1 or 2, wherein the X-ray powder diffraction pattern comprises reflections at 2-theta values of (3.9 ± 0.2) °, (6.3 ± 0.2) °, (7.9 ± 0.2) °, (13.4 ± 0.2) °, (15.1 ± 0.2) °, (25.4 ± 0.2) °, when measured at a temperature in the range of from 15 to 25 °C with Cu-Kalpha_{1,2} radiation having a wavelength of 0.15419 nm.
4. A process for preparing a crystalline hydrate form of afatinib dimaleate of formula (I) 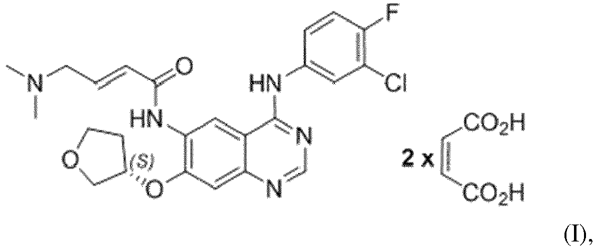 preferably the crystalline hydrate form of afatinib dimaleate of formula (I) according to any of embodiments 1 to 3, comprising
   (i) providing solid afatinib dimaleate of formula (I) in crystalline form 7 (having an X-ray powder diffraction pattern as described herein above);
   (ii) subjecting the non-dried solid state afatinib dimaleate provided in (i) to drying at a temperature in the range of from 15 to 30 °C at an absolute pressure of at most 500 mbar for 1 to 24 hours, obtaining a crystalline form of afatinib dimaleate of formula (I) having an X-ray powder diffraction pattern comprising reflections at at least four 2-theta values of (3.9 ± 0.2) °, (6.3 ± 0.2) °, (7.9 ± 0.2) °, (13.4 ± 0.2) °, (15.1 ± 0.2) °, (25.4 ± 0.2) °, when measured at a temperature in the range of from 15 to 25 °C with Cu-Kalpha_{1,2} radiation having a wavelength of 0.15419 nm.
5. The process of embodiment 4, wherein in (ii), the non-dried solid state afatinib dimaleate provided in (i) is subjected to drying at a temperature in the range of from 20 to 25 °C at an absolute pressure of at most 250 mbar, preferably of at most 100 mbar, for 6 to 24 hours, preferably for 8 to 16 hours.
6. The process of embodiment 4 or 5, wherein in (i), providing the non-dried solid afatinib dimaleate of formula (I) comprises
   (i-1) providing solid afatinib dimaleate of formula (I) in crystalline form 1 (having an X-ray powder diffraction pattern as described herein above);
   (i-2) dissolving the solid afatinib dimaleate of formula (I) provided in (i-1) in a solvent comprising a ketone, preferably comprising acetone, at a temperature of at least 20 °C;
   (i-3) cooling the solution obtained in (i-2) to a temperature of at most 15 °C, obtaining the solid afatinib dimaleate of formula (I) having an X-ray powder diffraction pattern comprising reflections at at least four 2-theta values of (5.1 ± 0.2) °, (10.1 ± 0.2) °, (13.9 ± 0.2) °, (15.2 ± 0.2) °, (20.4 ± 0.2) °, when measured at a temperature in the range of from 15 to 25 °C with Cu-Kalpha_{1,2} radiation having a wavelength of 0.15419 nm, in its mother liquor;
   (i-4) preferably separating the solid afatinib dimaleate of formula (I) obtained in (i-3) from its mother liquor.
7. The process of embodiment 6, wherein in (i-2), at least 99 weight-% of the solvent, preferably at least 99.5 weight of the solvent consist of acetone.
8. The process of embodiment 6 or 7, wherein in (i-2), the temperature is in the range of from 20 to 30 °C, preferably from 22.5 to 27.5 °C.
9. The process of any of embodiments 6 to 7, wherein in (i-3), the solution is cooled to a temperature in the range of from 5 to 15 °C, preferably from 7.5 to 12.5 °C.
10. The process of any of embodiments 6 to 9, wherein in (i-3), the solution is cooled at a cooling rate in the range of from 0.5 to 1.5 °C/min, preferably from 0.75 to 1.25 °C/min.
11. The process of any of embodiments 6 to 10, wherein the solid afatinib dimaleate of formula (I) obtained in (i-3) is separated from its mother liquor in (i-4), wherein the separating comprises subjecting the mother liquor comprising the solid afatinib dimaleate of formula (I) to filtration or centrifugation, preferably filtration, obtaining the non-dried afatinib dimaleate of formula (I) having an X-ray powder diffraction pattern comprising reflections at at least four 2-theta values of (5.1 ± 0.2) °, (10.1 ± 0.2) °, (13.9 ± 0.2) °, (15.2 ± 0.2) °, (20.4 ± 0.2) °, when measured at a temperature in the range of from 15 to 25 °C with Cu-Kalpha_{1,2} radiation having a wavelength of 0.15419 nm.
12. The process of any of embodiments 6 to 11, wherein in (i), providing the non-dried solid afatinib dimaleate of formula (I) comprises, preferably consists of
   (i-1) providing solid afatinib dimaleate of formula (I) in crystalline form 1 (having an X-ray powder diffraction pattern as described herein above);
   (i-2) dissolving the solid afatinib dimaleate of formula (I) provided in (i-1) in acetone at a temperature in the range of from 22.5 to 27.5 °C;
   (i-3) cooling the solution obtained in (i-2) to a temperature in the range of from 7.5 to 12.5 °C, obtaining the solid afatinib dimaleate of formula (I) having an X-ray powder diffraction pattern comprising reflections at at least four 2-theta values of (5.1 ± 0.2) °, (10.1 ± 0.2) °, (13.9 ± 0.2) °, (15.2 ± 0.2) °, (20.4 ± 0.2) °, when measured at a temperature in the range of from 15 to 25 °C with Cu-Kalpha_{1,2} radiation having a wavelength of 0.15419 nm, in its mother liquor;
   (i-4) separating the solid afatinib dimaleate of formula (I) obtained in (i-3) from its mother liquor by filtration.
13. The process of embodiment 4 or 5, wherein in (i), providing the non-dried solid afatinib dimaleate of formula (I) comprises
   (i-1) providing solid amorphous afatinib dimaleate of formula (I);
   (i-2) slurrying the solid amorphous afatinib dimaleate of formula (I) provided in (i-1) in a liquid medium comprising water and an alcohol, preferably a propanol, more preferably n-propanol, at a temperature in the range of from 15 to 35 °C, preferably from 20 to 25 °C;
   (i-3) subjecting the slurry obtained in (i-2) to a temperature cycle between a minimum temperature T₁ and a maximum temperature T₂, wherein T₁ is in the range of from 2 to 15 °C and T₂ is in the range of from 30 to 50 °C, obtaining the solid afatinib dimaleate of formula (I) having an X-ray powder diffraction pattern comprising reflections at at least four 2-theta values of (5.1 ± 0.2) °, (10.1 ± 0.2) °, (13.9 ± 0.2) °, (15.2 ± 0.2) °, (20.4 ± 0.2) °, when measured at a temperature in the range of from 15 to 25 °C with Cu-Kalpha_{1,2} radiation having a wavelength of 0.15419 nm, in the liquid medium;
   (i-4) preferably separating the solid afatinib dimaleate of formula (I) obtained in (i-3) from the liquid medium.
14. The process of embodiment 13, wherein in (i-2), from 65 to 95 weight-%, preferably from 75 to 85 weight-% of the liquid medium consist of the alcohol, preferably the propanol, more preferably n-propanol, and from 5 to 35 weight-%, preferably from 15 to 25 weight-% of the liquid medium consist of water.
15. The process of embodiment 13 or 14, wherein during the temperature cycle, the slurry is kept at T₁ for 0.5 to 10 h, preferably from 1 to 7.5 h, more preferably from 3 to 5 h, and at T₂ for 0.5 to 10 h, preferably from 1 to 7.5 h, more preferably from 3 to 5 h, wherein the temperature cycle is carried out for an overall time in the range of from 12 to 84 hours, preferably from 24 to 80 hours, more preferably from 48 to 76 hours, wherein, if T₁ and/or T₂ is realized more than once, the individual durations for which the slurry is kept at T₁ and/or T₂ can be the same or different from each other.
16. The process of any of embodiments 13 to 15, wherein in (i-3), T₁ is in the range of from 2 to 10 °C, preferably from 2 to 7.5 °C, and T₂ is in the range of from 35 to 45 °C, preferably from 37.5 to 42.5 °C, wherein, if T₁ and/or T₂ is realized more than once, the individual T₁ and/or T₂ can be the same or different from each other.
17. The process of any of embodiments 13 to 16, wherein during the temperature cycle, the heating rate from T₁ to T₂ in the range of from 0.5 to 1.5 °C/min, preferably from 0.75 to 1.25 °C/min and the cooling rate from T₂ to T₁ in the range of from 0.5 to 1.5 °C/min, preferably from 0.75 to 1.25 °C/min.
18. The process of any of embodiments 13 to 17, wherein the solid afatinib dimaleate of formula (I) obtained in (i-3) is separated from the liquid medium in (i-4), wherein the separating comprises subjecting the liquid medium comprising the solid afatinib dimaleate of formula (I) to filtration or centrifugation, preferably filtration, obtaining the non-dried afatinib dimaleate of formula (I) having an X-ray powder diffraction pattern comprising reflections at at least four 2-theta values of (5.1 ± 0.2) °, (10.1 ± 0.2) °, (13.9 ± 0.2) °, (15.2 ± 0.2) °, (20.4 ± 0.2) °, when measured at a temperature in the range of from 15 to 25 °C with Cu-Kalpha_{1,2} radiation having a wavelength of 0.15419 nm.
19. The process of any of embodiments 13 to 18, wherein in (i), providing the non-dried solid afatinib dimaleate of formula (I) comprises
   (i-1) providing solid amorphous afatinib dimaleate of formula (I);
   (i-2) slurrying the solid amorphous afatinib dimaleate of formula (I) provided in (i-1) in a liquid medium, wherein 75 to 85 weight-% of the liquid medium consist of n-propanol and from 15 to 25 weight-% of the liquid medium consist of water, at a temperature in the range of from 20 to 25 °C;
   (i-3) subjecting the slurry obtained in (i-2) to a temperature cycle between a minimum temperature T₁ and a maximum temperature T₂, wherein T₁ is in the range of from 2 to 7.5 °C and T₂ is in the range of from 37.5 to 42.5 °C, obtaining the solid afatinib dimaleate of formula (I) having an X-ray powder diffraction pattern comprising reflections at at least four 2-theta values of (5.1 ± 0.2) °, (10.1 ± 0.2) °, (13.9 ± 0.2) °, (15.2 ± 0.2) °, (20.4 ± 0.2) °, when measured at a temperature in the range of from 15 to 25 °C with Cu-Kalpha_{1,2} radiation having a wavelength of 0.15419 nm, in the liquid medium, wherein during the temperature cycle, the slurry is kept at T₁ for 3 to 5 h and at T₂ for 3 to 5 h, wherein the temperature cycle is carried out for an overall time in the range of from 48 to 76 hours, and wherein during the temperature cycle, the heating rate from T₁ to T₂ is in the range of from 0.75 to 1.25 °C/min and the cooling rate from T₂ to T₁ is in the range of from 0.75 to 1.25 °C/min;
   (i-4) separating the solid afatinib dimaleate of formula (I) obtained in (i-3) from the liquid medium by filtration.
20. A process for preparing the crystalline hydrate form of afatinib dimaleate of formula (I) according to any of embodiments 1 to 3, comprising
   (i) providing solid afatinib dimaleate of formula (I) in crystalline form 1 (having an X-ray powder diffraction pattern as described herein above);
   (ii) slurrying the solid afatinib dimaleate of formula (I) provided in (i) in a liquid medium comprising an nitrile, preferably acetonitrile, at a temperature in the range of from 15 to 35 °C, wherein preferably at least 99 weight-%, more preferably at least 99.5 weight-% of the liquid medium consist of acetonitrile;
   (iii) agitating, preferably stirring the slurry obtained in (ii) at a temperature in the range of from 40 to 60 °C, for 24 to 96 hours;
   (iv) separating the solid from the liquid medium, preferably by filtration or centrifigation, more preferably by filtration;
   (v) subjecting the solid obtained from (iv) to drying at a temperature in the range of from 15 to 30 °C.
21. The process of embodiment 20, wherein in (ii), the temperature is in the range of from 20 to 25 °.
22. The process of embodiment 21, wherein in (ii), the temperature is in the range of from 45 to 55 °C, preferably from 47.5 to 52.5 °C.
23. The process of any of embodiments 20 to 22, wherein in (iii), agitating, preferably stirring, is carried out for 48 to 84 h, preferably for 60 to 78 h.
24. The process of any of embodiments 20 to 23, wherein in (v), the temperature is in the range of from 20 to 25 °.
25. The process of any of embodiment 20 to 24, wherein in (v), the solid obtained from (i-4) is subjected to drying at an absolute pressure in the range of from 500 mbar to 2 bar, preferably from 750 mbar to 1.5 bar, more preferably at ambient pressure.
26. The process of any of embodiments 20 to 25, comprising
   (i) providing solid afatinib dimaleate of formula (I) in crystalline form 1 (having an X-ray powder diffraction pattern as described herein above);
   (ii) slurrying the solid afatinib dimaleate of formula (I) provided in (i) in acetonitrile at a temperature in the range of from 20 to 25 °C;
   (iii) agitating, preferably stirring the slurry obtained in (iii) at a temperature in the range of from 47.5 to 52.5 °C for 60 to 78 hours;
   (iv) separating the solid from the acetonitrile by filtration;
   (v) subjecting the solid obtained from (iv) to drying at a temperature in the range of from 20 to 25 °C at an absolute pressure in the range of from 5 mbar to 100 mbar.
27. A crystalline hydrate form of afatinib dimaleate of formula (I) 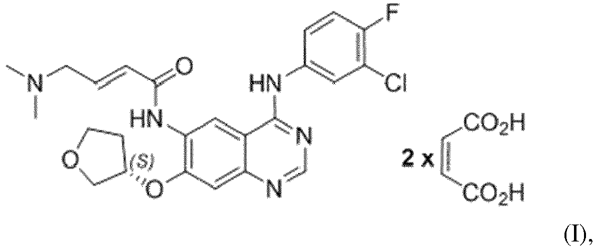 obtainable or obtained by a process according to any of embodiments 4 to 26, preferably according to embodiment 19 or embodiment 26.
28. A crystalline hydrate form of afatinib dimaleate of formula (I), also referred to herein as form 4, 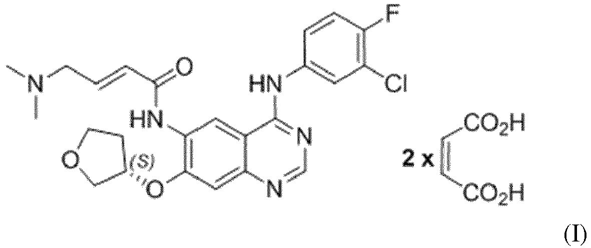 having an X-ray powder diffraction pattern comprising reflections at 2-theta values of (5.5 ± 0.2) °, (9.8 ± 0.2) °, (11.7 ± 0.2) °, (14.9 ± 0.2) °, when measured at a temperature in the range of from 15 to 25 °C with Cu-Kalpha_{1,2} radiation having a wavelength of 0.15419 nm.
29. The crystalline hydrate form of embodiment 28, wherein the X-ray powder diffraction pattern additionally comprises one or more reflections at 2-theta values of (6.1 ± 0.2) °, (25.6 ± 0.2) °, when measured at a temperature in the range of from 15 to 25 °C with Cu-Kalpha_{1,2} radiation having a wavelength of 0.15419 nm.
30. A process for preparing a crystalline hydrate form of afatinib dimaleate of formula (I) 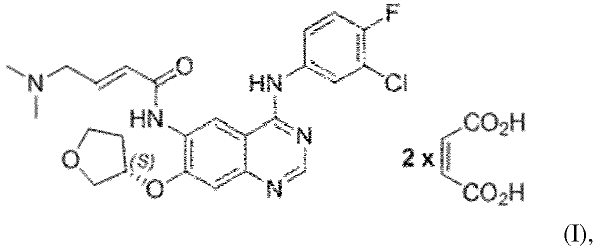 preferably the crystalline hydrate form of afatinib dimaleate of formula (I) according to embodiment 28 or 29, comprising
   (i) providing solid amorphous afatinib dimaleate of formula (I);
   (ii) slurrying the solid amorphous afatinib dimaleate of formula (I) provided in (i) in a liquid medium comprising an alcohol, preferably a propanol, more preferably n-propanol, at a temperature in the range of from 15 to 35 °C, preferably from 20 to 25 °C;
   (iii) subjecting the slurry obtained in (ii) to a temperature cycle between a minimum temperature T₁ and a maximum temperature T₂, wherein T₁ is in the range of from 2 to 15 °C and T₂ is in the range of from 30 to 50 °C, obtaining a crystalline form of afatinib dimaleate of formula (I) having an X-ray powder diffraction pattern comprising reflections at 2-theta values of (5.5 ± 0.2) °, (9.8 ± 0.2) °, (11.7 ± 0.2) °, (14.9 ± 0.2) °, when measured at a temperature in the range of from 15 to 25 °C with Cu-Kalpha_{1,2} radiation having a wavelength of 0.15419 nm, in the liquid medium;
   (iv) preferably separating the crystalline afatinib dimaleate of formula (I) obtained in (iii) from the liquid medium;
   (v) preferably drying the separated crystalline afatinib dimaleate of formula (I).
31. The process of embodiment 30, wherein in (ii), at least 99 weight-%, preferably at least 99.5 weight-% of the liquid medium consist of the alcohol, preferably the propanol, more preferably n-propanol.
32. The process of embodiment 30 or 31, wherein in (iii), during the temperature cycle, the slurry is kept at T₁ for 0.5 to 10 h, preferably from 1 to 7.5 h, more preferably from 3 to 5 h, and at T₂ for 0.5 to 10 h, preferably from 1 to 7.5 h, more preferably from 3 to 5 h, wherein the temperature cycle is carried out for an overall time in the range of from 12 to 168 hours, preferably from 24 to 144 hours, more preferably from 48 to 132 hours, wherein, if T₁ and/or T₂ is realized more than once, the individual durations for which the slurry is kept at T₁ and/or T₂ can be the same or different from each other.
33. The process of any of embodiments 30 to 32, wherein in (iii), T₁ is in the range of from 2 to 10 °C, preferably from 2 to 7.5 °C, and T₂ is in the range of from 35 to 45 °C, preferably from 37.5 to 42.5 °C, wherein, if T₁ and/or T₂ is realized more than once, the individual T₁ and/or T₂ can be the same or different from each other.
34. The process of any of embodiments 30 to 33, wherein in (iii), during the temperature cycle, the heating rate from T₁ to T₂ in the range of from 0.5 to 1.5 °C/min, preferably from 0.75 to 1.25 °C/min and the cooling rate from T₂ to T₁ in the range of from 0.5 to 1.5 °C/min, preferably from 0.75 to 1.25 °C/min.
35. The process of any of embodiments 30 to 34, wherein the crystalline afatinib dimaleate of formula (I) obtained in (iii) is separated from the liquid medium in (iv), wherein the separating comprises subjecting the liquid medium comprising the crystalline hydrate afatinib dimaleate of formula (I) to filtration or centrifugation, preferably filtration, obtaining the crystalline afatinib dimaleate of formula (I) having an X-ray powder diffraction pattern comprising reflections at 2-theta values of (5.5 ± 0.2) °, (9.8 ± 0.2) °, (11.7 ± 0.2) °, (14.9 ± 0.2) °, when measured at a temperature in the range of from 15 to 25 °C with Cu-Kalpha_{1,2} radiation having a wavelength of 0.15419 nm.
36. The process of any of embodiments 30 to 35, wherein the separated crystalline afatinib dimaleate of formula (I) obtained in (iv) is dried, wherein the drying is carried out at a temperature in the range of from 15 to 30 °C, preferably from 20 to 25 °C.
37. The process of any of embodiment 30 to 36, wherein in (v), the separated crystalline afatinib dimaleate of formula (I) obtained in (iv) is subjected to drying at an absolute pressure in the range of from 500 mbar to 2 bar, preferably from 750 mbar to 1.5 bar, more preferably at ambient pressure.
38. The process of any of embodiments 30 to 37, comprising
   (i) providing solid amorphous afatinib dimaleate of formula (I);
   (ii) slurrying the solid amorphous afatinib dimaleate of formula (I) provided in (i) in n-propanol, at a temperature in the range of from 20 to 25 °C;
   (iii) subjecting the slurry obtained in (ii) to a temperature cycle between a minimum temperature T₁ and a maximum temperature T₂, wherein T₁ is in the range of from 2 to 7.5 °C and T₂ is in the range of from 37.5 to 42.5 °C, obtaining a crystalline afatinib dimaleate of formula (I) having an X-ray powder diffraction pattern comprising reflections at 2-theta values of (5.5 ± 0.2) °, (9.8 ± 0.2) °, (11.7 ± 0.2) °, (14.9 ± 0.2) °, when measured at a temperature in the range of from 15 to 25 °C with Cu-Kalpha_{1,2} radiation having a wavelength of 0.15419 nm, in the liquid medium, wherein during the temperature cycle, the slurry is kept at T₁ for 3 to 5 h and at T₂ for 3 to 5 h, wherein the temperature cycle is carried out for an overall time in the range of from 48 to 76 hours, and wherein during the temperature cycle, the heating rate from T₁ to T₂ is in the range of from 0.75 to 1.25 °C/min and the cooling rate from T₂ to T₁ is in the range of from 0.75 to 1.25 °C/min;
   (iv) separating the crystalline afatinib dimaleate of formula (I) obtained in (iii) from the n-propanol;
   (v) drying the separated crystalline afatinib dimaleate of formula (I) at a temperature in the range of from 20 to 25 °C at an absolute pressure in the range of from 5 mbar to 100 mbar.
39. A crystalline hydrate form of afatinib dimaleate of formula (I) 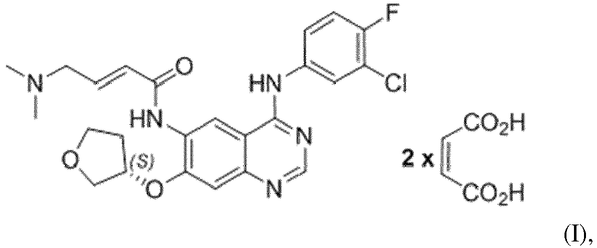 obtainable or obtained by a process according to any of embodiments 28 to 38, preferably according to embodiment 38.
40. A crystalline hydrate form of afatinib dimaleate of formula (I), also referred to herein as form 3, 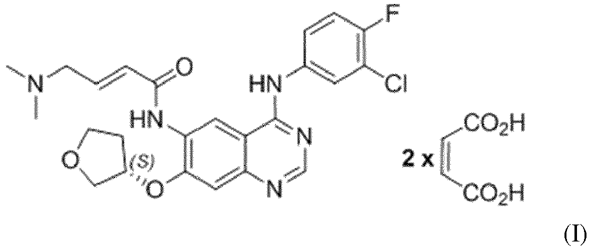 having an X-ray powder diffraction pattern comprising reflections at 2-theta values of (5.3 ± 0.2) °, (10.2 ± 0.2) °, (10.7 ± 0.2) °, when measured at a temperature in the range of from 15 to 25 °C with Cu-Kalpha_{1,2} radiation having a wavelength of 0.15419 nm.
41. A crystalline hydrate form of afatinib dimaleate of formula (I), also referred to herein as form 7, 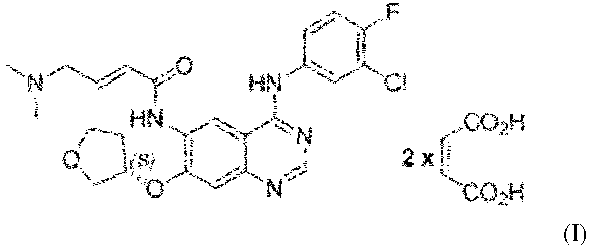 having an X-ray powder diffraction pattern comprising reflections at at least four 2-theta values of (5.1 ± 0.2) °, (10.1 ± 0.2) °, (13.9 ± 0.2) °, (15.2 ± 0.2) °, (20.4 ± 0.2) °, when measured at a temperature in the range of from 15 to 25 °C with Cu-Kalpha_{1,2} radiation having a wavelength of 0.15419 nm.
42. The crystalline hydrate form of embodiment 41, wherein the X-ray powder diffraction pattern comprises reflections at 2-theta values of (5.1 ± 0.2) °, (10.1 ± 0.2) °, (13.9 ± 0.2) °, (15.2 ± 0.2) °, (20.4 ± 0.2) °, when measured at a temperature in the range of from 15 to 25 °C with Cu-Kalpha_{1,2} radiation having a wavelength of 0.15419 nm.
43. A pharmaceutical composition, preferably for treating cancer in a mammal, preferably in a human, wherein the cancer is preferably lung cancer, more preferably non-small lung cancer, said pharmaceutical composition comprising a crystalline hydrate form of afatinib dimaleate of formula (I) 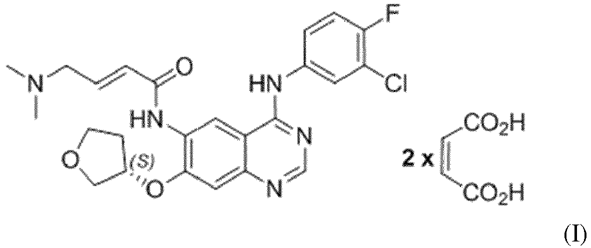 according to any of embodiments 1 to 3 or 27, or according to embodiment 28 or 29 or 39, or according to embodiment 40, or according to embodiment 41 or 42, or a mixture of two or more thereof, preferably according to any of embodiments 1 to 3 or 27, said pharmaceutical composition optionally additionally comprising at least one pharmaceutically acceptable excipient.
44. The pharmaceutical composition of embodiment 72, which is a solid pharmaceutical composition.

The following non-limiting examples are illustrative for the disclosure.

### Examples

### Reference Example 1: Methods for the Determination of Chemical and Physical Properties

### 1.1 Determination of X-ray Powder Diffraction (XRPD) Patterns

One of ordinary skill in the art will appreciate that an X-ray diffraction pattern may be obtained with a measurement error that is dependent upon the measurement conditions employed. In particular, it is generally known that intensities in an X-ray diffraction pattern may fluctuate depending upon measurement conditions employed. It should be further understood that relative intensities may also vary depending upon experimental conditions and, accordingly, the exact order of intensity should not be taken into account. Additionally, a measurement error of diffraction angle for a conventional X-ray diffraction pattern is typically about 5% or less, and such degree of measurement error should be taken into account as pertaining to the aforementioned diffraction angles. Consequently, it is to be understood that the crystal forms of the instant invention are not limited to the crystal forms that provide X-ray diffraction patterns completely identical to the X-ray diffraction patterns depicted in the accompanying Figures disclosed herein. Any crystal forms that provide X- ray diffraction patterns substantially identical to those disclosed in the accompanying Figures fall within the scope of the present invention. The ability to ascertain substantial identities of X-ray diffraction patterns is within the purview of one of ordinary skill in the art.

XRPD analysis was carried out on a Siemens D5000, scanning the samples between 3° and 30° 2Theta. For small sample amounts, the material was gently compressed onto a glass slide, fitted into an XRPD sample holder.

| | |
|---|---|
| Raw Data Origin | Siemens-binary V2 (.RAW) |
| Start Position [° 2Theta] | 3.0000 |
| End Position [° 2Theta] | 30.000 |
| Step Size [° 2Theta] | 0.0200 |
| *Scan Step Time [s] | 1 |
| Scan Type | Continuous |
| Offset [° 2Theta] | 0.0000 |
| Divergence Slit Type | Fixed |
| Divergence Slit Size [°] | 2.0000 |
| Specimen Length [mm] | various |
| Receiving Slit Size [mm] | 0.2000 |
| Measurement Temperature [°C] | 20.00 |
| Anode Material | Cu |
| K-Alphal/2 [Angstrom] | 1.5419 |
| K-Beta [Angstrom] | 1.39225 |
| K-A2 / K-A1 Ratio | 0.50000 (nominal) |
| Generator Settings | 40 mA, 40 kV |
| Diffractometer Type | d5000 |
| Goniometer Radius [mm] | 217.50 |
| Incident Beam Monochromator | No |
| Diffracted Beam Monochromator | (Graphite) |
| Spinning | No |

For some experiments, in order to improve the signal to noise ratio, the scan step time was increased to 5 or 12 seconds and/or a zero background slide was employed.

### 1.2 Determination of the Water Content of the Crystalline Forms by Karl Fischer Titration

Initially a blank sample containing methanol only was analysed by KF (Mettler Toledo C30 Compact Titrator) to determine the blank water content of the dissolution solvent before sample analysis. Approximately 10 mg of solid material was accurately weighed into a vial. The material was then dissolved in methanol, the mass of which was recorded. The resultant solution was then manually introduced into the titration cell of a Mettler Toledo C30 Compact Titrator. The water content was calculated as a percentage using an external dissolution method and the data printed.

### 1.3 Differential Scanning Calorimetry (DSC)

The DSC cell/sample chamber was purged with 100 ml/min of ultra-high purity nitrogen gas. The instrument was calibrated with high purity indium. The accuracy of the measured sample temperature with this method is within about ± 1 °C, and the heat of fusion can be measured within a relative error of about ± 5 %.

Differential scanning calorimetry was conducted on an Seiko Exstar DSC6200 instrument. For each crystalline form as follows: approximately 5 mg of material was weighed into an aluminium DSC pan and sealed non-hermetically with a pierced aluminium lid. The sample pan was then loaded into a Seiko DSC6200 (equipped with a cooler). The sample and reference were heated to about 270 °C (unless otherwise stated) at a scan rate of 10 °C/min, and the resulting heat flow response monitored. The heat flow, which was normalized by sample weight, was plotted versus the measured sample temperature. The data were reported in units of watts/gram ("W/g"). The plot was made with the endothermic peaks pointing down. The endothermic melt peak (melting point) was evaluated for extrapolated onset temperature.

### 1.4 Thermogravimetric Analysis (TGA)

The TGA instruments used to test the crystalline forms was a Seiko Exstar TG/DTA6200. Approximately 5 mg of material was weighed into an open aluminium pan and loaded into a simultaneous thermogravimetric/differential thermal analyser (TG/DTA) and held at room temperature. The sample was then heated at a rate of 10 °C/min from 25 °C to 300 °C (unless otherwise stated) during which time the change in sample weight was recorded along with any differential thermal events (DTA). Nitrogen was used as the purge gas, at a flow rate of 100 cm³/min.

### 1.5 High Performance Liquid Chromatography-Ultraviolet Detection (HPLC-UV)

To determine concentration, solutions were diluted in methanol; to determine purity, 2.7 mg material was dissolved in 1 mL methanol. The conditions listed below were then used.

| | |
|---|---|
| Instrument: | Agilent 1100 |
| Column: | Waters Symmetry C18 5micron 150x3.9 mm column |
| Column Temperature: | 25 °C |
| Autosampler Temperature: | Not controlled |
| UV wavelength: | 254 nm |
| Injection Volume: | 3 microL |
| Flow Rate: | 1.2 mL/min |
| Mobile Phase A: | 0.01 % formic acid in water |
| Mobile Phase B: | 0.01 % formic acid in ACN |

Gradient program:

| Time (minutes) | Solvent B [%] |
|---|---|
| 0.0 | 20 |
| 20.0 | 80 |
| 22.0 | 80 |
| 22.5 | 95 |
| 25.0 | 95 |
| 25.3 | 20 |
| 30.0 | 20 |

### Reference Example 2: Preparation of Amorphous Afatinib Dimaleate

With regard to a process for the preparation of crystalline material of afatinib dimaleate, reference can be made, for example, to WO 2005/037824, WO 2007/085638, WO 2012/121764, and WO 2013/052157. One possible way to provide amorphous material of afatinib dimaleate is to provide crystalline material of afatinib dimaleate (such as form 1 as described in example 3 of WO 2005/037824) and to covert this material into amorphous material. Methods of converting crystalline material into amorphous material is well known to the person skilled in the art (see e.g. grounding in a ball mill as described below). Amorphous material of afatinib dimaleate is desired to remove any 'memory' that the material might have of any crystalline form.

The provided crystalline material of afatinib dimaleate was ground in a ball mill. The material was ground at 50 Hz for ca. 1 hour (500 mg at a time in each jar). After grinding, the material was analysed by PLM and XRPD to confirm that amorphous material had been obtained.

### Reference Example 3: Preparation of Crystalline Form 7 of Afatinib Dimaleate

5 mL of 2-propanol (80 %) : water (20 %) were added to 500 mg of the amorphous form of afatinib dimaleate according to Reference Example 2 to form a slurry. The slurry was temperature cycled between 5 °C and 40 °C (same cycling profile as Form 3 as described in Example 3 below) for about 3 days. The sample was filtered and was not dried prior to characterization by XRPD as described in Reference Example 1.1 above.

### Reference Example 4: Preparation of Crystalline Form 7 of Afatinib Dimaleate

Approximately 500 mg afatinib dimaleate material of crystalline form 1 according to Reference Example 5 below was weighed into a vial and 300 microL acetone aw 0.91 were added at room temperature to make a slurry. The sample was observed to be too thick to stir, so an additional 40 microL solvent were added. The slurry was then stirred at about 25 °C for about 24 hours. A further 270 microL acetone aw 0.91 were then added, the slurry was stirred at about 25 °C for about a further 24 hours after which the sample was observed to undergo complete dissolution. It was cooled to 10 °C and the resultant solid material was analysed by XRPD as described in Reference Example 1.1 above. The XRPD pattern is shown in Figure 1. The corresponding peak table is as follows:

| **Pos. [°2θ]** | **Height [cts]** | **Rel. Int. [%]** |
|---|---|---|
| 5.1327 | 1614.88 | 100.00 |
| 5.8252 | 138.14 | 8.55 |
| 7.5781 | 22.98 | 1.42 |
| 8.1153 | 35.42 | 2.19 |
| 9.4678 | 88.01 | 5.45 |
| 10.1349 | 540.23 | 33.45 |
| 10.9041 | 82.64 | 5.12 |
| 11.3694 | 85.96 | 5.32 |
| 13.0391 | 70.33 | 4.36 |
| 13.8949 | 120.09 | 7.44 |
| 15.2535 | 245.50 | 15.20 |
| 16.9619 | 45.90 | 2.84 |
| 18.9443 | 42.83 | 2.65 |
| 19.7974 | 125.39 | 7.76 |
| 20.4196 | 945.93 | 58.58 |
| 21.2151 | 99.95 | 6.19 |
| 22.2226 | 36.87 | 2.28 |
| 23.7038 | 22.50 | 1.39 |
| 25.6972 | 166.76 | 10.33 |

### Reference Example 5: Preparation of Afatinib Dimaleate Form 1

With regard to a process for the preparation of crystalline form 1 of afatinib dimaleate, reference can be made, for example, to WO 2005/037824, WO 2007/085638, WO 2012/121764, and WO 2013/052157. Solid afatinib dimaleate of formula (I) in crystalline form 1 has an X-ray powder diffraction pattern as described in WO 2005/037824 (see e.g. Figure 1), which form is referred to herein also as "form A". One possible way to provide afatinib dimaleate in crystalline form 1 (i.e. form A) is described in example 3 of WO 2005/037824.

### Reference Example 6: Preparation of Crystalline Form 7 of Afatinib Dimaleate

Afatinib dimaleate (500mg, Form 1) was weighed into a vial and 314 µL 2-propanol aw 0.9 was added at 50°C. Most of the material dissolved and the solution was stirred for ca. 24 hours. The sample was then allowed to cool to ambient and the resultant solid material was analysed by XRPD as described in Reference Example 1.1 above.

### Reference Example 7: Preparation of Crystalline Form 7 of Afatinib Dimaleate

Afatinib dimaleate (500mg, Form 1) was weighed into a vial and 583 µL 2-propanol aw 0.88 was added at room temperature to make a slurry. On complete dissolution, an additional 100 mg of material was added. The slurry was then stirred at ca. 25°C for 24. A further 110 µL 2-propanol aw 0.88 was then added, the slurry was stirred at ca. 25°C for a further ca. 24 hours. Finally the slurry was warmed to 50°C until complete dissolution was observed. The sample was then cooled to 10°C and the resultant solid material was analysed by XRPD.

### Example 1: Preparation of Crystalline Form 12 of Afatinib Dimaleate from Crystalline Form 7

Form 7 obtained as described above (e.g. according to Reference Example 3) was subjected to drying under vacuum 22 °C for a period of 12 h. The resulting material was analysed by XRPD as described in Reference Example 1.1 above. The XRPD pattern is shown in Figure 2. The corresponding peak table is as follows:

| **Pos. [°2θ]** | **Height [cts]** | **Rel. Int. [%]** | | **Pos. [°2θ]** | **Height [cts]** | **Rel. Int. [%]** |
|---|---|---|---|---|---|---|
| 3.9382 | 448.06 | 12.96 | | 20.4560 | 399.85 | 11.56 |
| 5.5037 | 3457.53 | 100.00 | | 21.0312 | 256.74 | 7.43 |
| 5.6051 | 1982.88 | 57.35 | | 21.7349 | 242.15 | 7.00 |
| 6.1779 | 1007.57 | 29.14 | | 22.1830 | 286.33 | 8.28 |
| 6.2529 | 978.57 | 28.30 | | 22.7625 | 297.54 | 8.61 |
| 7.8981 | 60.06 | 1.74 | | 23.3906 | 156.48 | 4.53 |
| 8.8632 | 684.78 | 19.81 | | 23.8762 | 218.35 | 6.32 |
| 9.9359 | 142.13 | 4.11 | | 24.8524 | 553.22 | 16.00 |
| 11.0464 | 448.09 | 12.96 | | 25.3827 | 1482.83 | 42.89 |
| 11.2307 | 891.04 | 25.77 | | 25.6165 | 1274.08 | 36.85 |
| 11.3801 | 746.25 | 21.58 | | 26.0954 | 927.29 | 26.82 |
| 11.5751 | 367.84 | 10.64 | | 26.6031 | 1046.94 | 30.28 |
| 12.5243 | 725.49 | 20.98 | | 27.0710 | 489.46 | 14.16 |
| 13.4232 | 429.20 | 12.41 | | 27.7586 | 311.35 | 9.00 |
| 14.2757 | 337.67 | 9.77 | | 28.3717 | 175.31 | 5.07 |
| 14.5512 | 263.18 | 7.61 | | 29.0514 | 137.03 | 3.96 |
| 14.8659 | 367.23 | 10.62 | | | | |
| 15.0993 | 459.51 | 13.29 | | | | |
| 16.1070 | 141.44 | 4.09 | | | | |
| 16.8427 | 246.68 | 7.13 | | | | |
| 17.7021 | 314.22 | 9.09 | | | | |
| 17.9924 | 329.02 | 9.52 | | | | |
| 18.6189 | 400.22 | 11.58 | | | | |
| 18.8438 | 514.87 | 14.89 | | | | |
| 19.4762 | 317.12 | 9.17 | | | | |
| 19.8704 | 391.04 | 11.31 | | | | |

The water content of the crystalline hydrate form obtained, determined according to the method as described in Reference Example 1.2, was 4.9 weight-% based on the total weight of the crystalline compound.

The TG/DTA curves of the crystalline form 12 is shown in Figure 5, the respective DSC curve in Figure 6.

Stability tests at 40 °C/75 % relative humidity; at ambient conditions; and at 25°C / 60% relative humidity with post HPLC analysis at time points 7, 14 and 30 days showed the purity to remain relatively consistent for all samples. Post XRPD analysis showed all samples to be consistent with Form 12, with a slight loss in crystallinity for the 40 °C/75 % relative humidity samples.

### Example 2: Preparation of Crystalline Form 12 of Afatinib Dimaleate from Crystalline Form 1

200 mg of crystalline form 1 of afatinib dimaleate prepared according to Reference Example 5 was weighed into a vial, and 500 microL of acetonitrile were added. The resulting slurry was stirred at 50 °C for 72 h, filtered, and dried at ambient temperature. The resulting material was analysed by XRPD as described in Reference Example 1.1 above. The XRPD pattern showed crystalline form 12 of afatinib dimaleate.

### Example 3: Preparation of Crystalline Form 3 of Afatinib Dimaleate from the Amorphous Form

1.5 mL of acetone (50 %) : Water (50 %) were added to 1000 mg of amorphous form of afatinib dimaleate prepared according to Reference Example 2 to form a slurry. The slurry was temperature cycled between 5 °C and 40 °C (slurry was held at 40 °C for 4 hours followed by a hold at 5 °C for 4 hours, the cooling/heating rate after the 4 hour hold periods was about 1 °C/min) for about 3 days. The sample was filtered and allowed to dry at ambient temperature prior to characterization. XRPD analysis carried out according to Reference Example 1.1. The XRPD pattern is shown in Figure 3. The corresponding peak table is as follows:

| **Pos. [°2θ]** | **Height [cts]** | **Rel. Int. [%]** |
|---|---|---|
| 5.2525 | 3110.63 | 100.00 |
| 10.1832 | 89.83 | 2.89 |
| 10.7146 | 542.81 | 17.45 |
| 16.4587 | 26.44 | 0.85 |
| 20.6729 | 60.81 | 1.96 |
| 21.7496 | 330.27 | 10.62 |
| 27.4583 | 36.41 | 1.17 |

The water content of the crystalline hydrate form obtained, determined according to the method as described in Reference Example 1.2, was 7.3 weight-% based on the total weight of the crystalline compound. HPLC analysis indicated a purity of about 94.6% at 40°C/75% RH, about 76% at 80°C and about 96.4% at ambient. No change in the polymorphic form was observed after stability tests at various conditions.

### Example 4: Preparation of Crystalline Form 4 of Afatinib Dimaleate from the Amorphous Form

10 mL of 2-propanol were added to 500 mg of the amorphous form of afatinib dimaleate according to Reference Example 2 to form a slurry. The slurry was temperature cycled between 5 °C and 40 °C (same cycling profile as for form 3 according to Example 3) for about 3 days, followed by a further 2 days in attempts to improve the crystallinity. The sample was then filtered and allowed to dry at ambient temperature prior to characterization. XRPD analysis carried out according to Reference Example 1.1. The XRPD pattern is shown in Figure 4. The corresponding peak table is as follows:

| **Pos. [°2θ]** | **Height [cts]** | **Rel. Int. [%]** |
|---|---|---|
| 5.4843 | 1949.86 | 100.00 |
| 6.1429 | 453.89 | 23.28 |
| 8.6560 | 60.60 | 3.11 |
| 9.8804 | 81.53 | 4.18 |
| 11.0212 | 266.29 | 13.66 |
| 11.3619 | 464.84 | 23.84 |
| 11.6776 | 54.74 | 2.81 |
| 13.0415 | 105.02 | 5.39 |
| 14.8408 | 127.33 | 6.53 |
| 16.1010 | 107.79 | 5.53 |
| 16.6961 | 118.24 | 6.06 |
| 17.6705 | 223.00 | 11.44 |
| 19.7008 | 159.34 | 8.17 |
| 20.1490 | 208.88 | 10.71 |
| 21.1068 | 106.72 | 5.47 |
| 22.1404 | 241.91 | 12.41 |
| 22.8484 | 112.47 | 5.77 |
| 23.4889 | 76.27 | 3.91 |
| 25.5922 | 868.40 | 44.54 |
| 26.1474 | 494.86 | 25.38 |
| 26.9704 | 194.84 | 9.99 |
| 27.9529 | 174.02 | 8.93 |
| 29.0890 | 98.17 | 5.03 |

The water content of the crystalline hydrate form obtained, determined according to the method as described in Reference Example 1.2, was 8.0 weight-% based on the total weight of the crystalline compound. HPLC analysis indicated a purity of about 96.5% at 40°C/75% RH, about 81.6% at 80°C and about 98.9% at ambient conditions. No change in the polymorphic form was observed after stability tests at the various conditions.

### Results of the Examples

| **Analysis** | **Form 1** | **Form 3** | **Form 4** | **Form 12** |
|---|---|---|---|---|
| | | | | |
| **Post-DVS XRPD** | Conversion to Form 4 | Unchanged | Unchanged | Unchanged |
| **Karl Fisher (water in weight-%)** | N/A | 7.3% | 8.0% | 4.9% |
| **Stability: ambient** | Unchanged, purity 98.5% | Unchanged, purity 96.4% | Unchanged, purity 98.9% | Unchanged, purity 96.9% |

Compared to the anhydrous form 1, all forms 3, 4, and 12 exhibit essentially the same stability at ambient conditions while Post-DVS measurements even showed that form 1 is converted to hydrate form 4 while hydrate forms 3, 4, and 12 remain unchanged. Thus, against the prejudice of the prior art according to which the anhydrous form A (form 1) of the afatinib dimaleate would be very much preferred for processing into tablets since hydrolytic decomposition of the active agent is a critical issue and the presence of water, e.g. as crystal water in hydrate forms, should thus be avoided in pharmaceutical tablet formulations, and that the hydrate forms of afatinib would therefore not be suitable for the production of the active agent to be processed into final solid formulations, the present inventors found that the presence of water in specific hydrates has no negative influence on the stability of these hydrates, and that, therefore, these hydrates can be processed into final solid formulations.

### Example 5: Pharmaceutical compositions

Pharmaceutical compositions comprising the crystalline hydrate forms of afatinib dimaleate of formula (I) are prepared according to WO 2009/147238 and WO 2011/003853 (which are incorporated herein with reference).

## Claims

1. A crystalline hydrate form of afatinib dimaleate of formula (I) 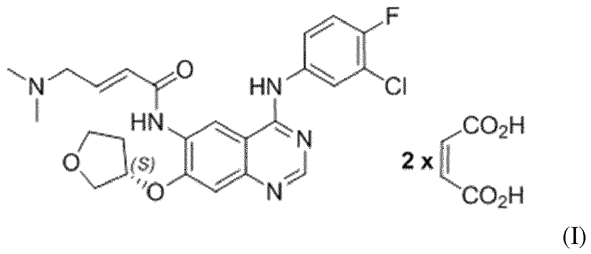 having an X-ray powder diffraction pattern comprising reflections at at least four 2-theta values of (3.9 ± 0.2) °, (6.3 ± 0.2) °, (7.9 ± 0.2) °, (13.4 ± 0.2) °, (15.1 ± 0.2) °, (25.4 ± 0.2) °, when measured at a temperature in the range of from 15 to 25 °C with Cu-Kalpha_{1,2} radiation having a wavelength of 0.15419 nm.

2. The crystalline hydrate form of claim 1, wherein the X-ray powder diffraction pattern comprises reflections at at least five 2-theta values of (3.9 ± 0.2) °, (6.3 ± 0.2) °, (7.9 ± 0.2) °, (13.4 ± 0.2) °, (15.1 ± 0.2) °, (25.4 ± 0.2) °, when measured at a temperature in the range of from 15 to 25 °C with Cu-Kalpha_{1,2} radiation having a wavelength of 0.15419 nm.

3. The crystalline hydrate form of claim 1 or 2, wherein the X-ray powder diffraction pattern comprises reflections at 2-theta values of (3.9 ± 0.2) °, (6.3 ± 0.2) °, (7.9 ± 0.2) °, (13.4 ± 0.2) °, (15.1 ± 0.2) °, (25.4 ± 0.2) °, when measured at a temperature in the range of from 15 to 25 °C with Cu-Kalpha_{1,2} radiation having a wavelength of 0.15419 nm.

4. A process for preparing the crystalline hydrate form of afatinib dimaleate of formula (I) according to any of claims 1 to 3, comprising
(i) providing solid afatinib dimaleate of formula (I) in crystalline form 7 having an X-ray powder diffraction pattern comprising reflections at at least four 2-theta values of (5.1 ± 0.2) °, (10.1 ± 0.2) °, (13.9 ± 0.2) °, (15.2 ± 0.2) °, (20.4 ± 0.2) °, when measured at a temperature in the range of from 15 to 25 °C with Cu-Kalpha_{1,2} radiation having a wavelength of 0.15419 nm;
(ii) subjecting the non-dried solid state afatinib dimaleate provided in (i) to drying at a temperature in the range of from 15 to 30 °C at an absolute pressure of at most 500 mbar for 1 to 24 hours, obtaining the crystalline hydrate form of afatinib dimaleate of formula (I) according to any of claims 1 to 3.

5. The process of claim 4, wherein in (i), providing the non-dried solid afatinib dimaleate of formula (I) comprises
(i-1) providing solid afatinib dimaleate of formula (I) in crystalline form 1;
(i-2) dissolving the solid afatinib dimaleate of formula (I) provided in (i-1) in a solvent comprising a ketone, preferably comprising acetone, at a temperature of at least 20 °C;
(i-3) cooling the solution obtained in (i-2) to a temperature of at most 15 °C, obtaining the solid afatinib dimaleate of formula (I) having an X-ray powder diffraction pattern comprising reflections at at least four 2-theta values of (5.1 ± 0.2) °, (10.1 ± 0.2) °, (13.9 ± 0.2) °, (15.2 ± 0.2) °, (20.4 ± 0.2) °, when measured at a temperature in the range of from 15 to 25 °C with Cu-Kalpha_{1,2} radiation having a wavelength of 0.15419 nm, in its mother liquor;
(i-4) preferably separating the solid afatinib dimaleate of formula (I) obtained in (i-3) from its mother liquor.

6. The process of claim 5, wherein in (i-2), at least 99 weight-% of the solvent, preferably at least 99.5 weight of the solvent consist of acetone.

7. The process of of claim 4, wherein in (i), providing the non-dried solid afatinib dimaleate of formula (I) comprises
(i-1) providing solid amorphous afatinib dimaleate of formula (I);
(i-2) slurrying the solid amorphous afatinib dimaleate of formula (I) provided in (i-1) in a liquid medium comprising water and an alcohol, preferably a propanol, more preferably n-propanol, at a temperature in the range of from 15 to 35 °C;
(i-3) subjecting the slurry obtained in (i-2) to a temperature cycle between a minimum temperature T₁ and a maximum temperature T₂, wherein T₁ is in the range of from 2 to 15 °C and T₂ is in the range of from 30 to 50 °C, obtaining the solid afatinib dimaleate of formula (I) having an X-ray powder diffraction pattern comprising reflections at at least four 2-theta values of (5.1 ± 0.2) °, (10.1 ± 0.2) °, (13.9 ± 0.2) °, (15.2 ± 0.2) °, (20.4 ± 0.2) °, when measured at a temperature in the range of from 15 to 25 °C with Cu-Kalpha_{1,2} radiation having a wavelength of 0.15419 nm, in the liquid medium;
(i-4) preferably separating the solid afatinib dimaleate of formula (I) obtained in (i-3) from the liquid medium.

8. The process of claim 7, wherein in (i-2), from 65 to 95 weight-%, preferably from 75 to 85 weight-% of the liquid medium consist of the alcohol, preferably the propanol, more preferably n-propanol, and from 5 to 35 weight-%, preferably from 15 to 25 weight-% of the liquid medium consist of water.

9. A process for preparing the crystalline hydrate form of afatinib dimaleate of formula (I) according to any of claims 1 to 3, comprising
(i) providing solid afatinib dimaleate of formula (I) in crystalline form 1;
(ii) slurrying the solid afatinib dimaleate of formula (I) provided in (i) in a liquid medium comprising an nitrile, preferably acetonitrile, at a temperature in the range of from 15 to 35 °C;
(iii) agitating, preferably stirring the slurry obtained in (ii) at a temperature in the range of from 40 to 60 °C, for 24 to 96 hours;
(iv) separating the solid from the liquid medium, preferably by filtration or centrifigation, more preferably by filtration;
(v) subjecting the solid obtained from (iv) to drying at a temperature in the range of from 15 to 30 °C.

10. The process of claim 9, wherein at least 99 weight-%, preferably at least 99.5 weight-% of the liquid medium consist of acetonitrile.

11. A crystalline hydrate form of afatinib dimaleate of formula (I) 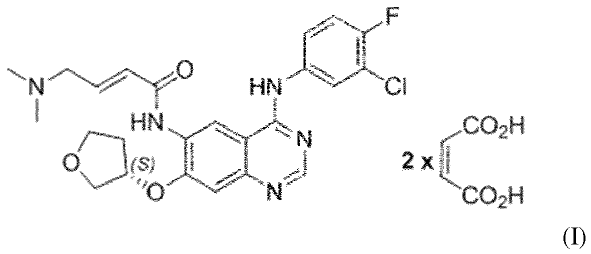 having an X-ray powder diffraction pattern comprising reflections at 2-theta values of (5.5 ± 0.2) °, (9.8 ± 0.2) °, (11.7 ± 0.2) °, (14.9 ± 0.2) °, when measured at a temperature in the range of from 15 to 25 °C with Cu-Kalpha_{1,2} radiation having a wavelength of 0.15419 nm.

12. The crystalline form of claim 11, wherein the X-ray powder diffraction pattern additionally comprises one or more reflections at 2-theta values of (6.1 ± 0.2) °, (25.6 ± 0.2) °, when measured at a temperature in the range of from 15 to 25 °C with Cu-Kalpha_{1,2} radiation having a wavelength of 0.15419 nm.

13. A process for preparing the crystalline form of afatinib dimaleate of formula (I) according to claim 11 or 12, comprising
(i) providing solid amorphous afatinib dimaleate of formula (I);
(ii) slurrying the solid amorphous afatinib dimaleate of formula (I) provided in (i) in a liquid medium comprising an alcohol, preferably a propanol, more preferably n-propanol, at a temperature in the range of from 15 to 35 °C, preferably from 20 to 25 °C;
(iii) subjecting the slurry obtained in (ii) to a temperature cycle between a minimum temperature T₁ and a maximum temperature T₂, wherein T₁ is in the range of from 2 to 15 °C and T₂ is in the range of from 30 to 50 °C, obtaining a crystalline form of afatinib dimaleate of formula (I) having an X-ray powder diffraction pattern comprising reflections at 2-theta values of (5.5 ± 0.2) °, (9.8 ± 0.2) °, (11.7 ± 0.2) °, (14.9 ± 0.2) °, when measured at a temperature in the range of from 15 to 25 °C with Cu-Kalpha_{1,2} radiation having a wavelength of 0.15419 nm, in the liquid medium;
(iv) preferably separating the crystalline afatinib dimaleate of formula (I) obtained in (iii) from the liquid medium;
(v) preferably drying the separated crystalline afatinib dimaleate of formula (I).

14. The process of claim 13, wherein in (ii), at least 99 weight-%, preferably at least 99.5 weight-% of the liquid medium consist of the alcohol, preferably the propanol, more preferably n-propanol.

15. A pharmaceutical composition for treating lung cancer in a human, comprising the crystalline hydrate form of afatinib dimaleate of formula (I) according to any of claims 1 to 3 or according to claim 11 or 12 or a mixture thereof, preferably comprising the crystalline hydrate form of afatinib dimaleate of formula (I) according to any of claims 1 to 3, said pharmaceutical composition optionally additionally comprising at least one pharmaceutically acceptable excipient, wherein said pharmaceutical composition is preferably a solid pharmaceutical composition.
